(19) Europäisches Patentamt
European Patent Office
Office européen des brevets

(11) Veröffentlichungsnummer : **0 360 091 B1**

(12) **EUROPÄISCHE PATENTSCHRIFT**

(45) Veröffentlichungstag der Patentschrift : **02.09.92 Patentblatt 92/36**

(51) Int. Cl.[5] : **C07C 45/51**, C07C 47/232

(21) Anmeldenummer : **89116611.8**

(22) Anmeldetag : **08.09.89**

# (54) Verfahren zur Herstellung von alkylsubstituierten Zimtaldehyden.

(30) Priorität : **17.09.88 DE 3831713**

(43) Veröffentlichungstag der Anmeldung : **28.03.90 Patentblatt 90/13**

(45) Bekanntmachung des Hinweises auf die Patenterteilung : **02.09.92 Patentblatt 92/36**

(84) Benannte Vertragsstaaten : **CH DE FR GB LI NL**

(56) Entgegenhaltungen : **GB-A- 267 954**

(56) Entgegenhaltungen : **ANN. CHIM., 1967, Seiten 243-249; M. BARRELLE et al.: "Comportement en milieu acide d'alcools secondaires alfa-acétyléniquesv-rais"**
**J.A.C.S., Band 70, 1948, Seite 3953; W.S. MacGREGOR: "Rearrangement of phenylethynylcarbinol"**

(73) Patentinhaber : **BASF Aktiengesellschaft**
**Carl-Bosch-Strasse 38**
**W-6700 Ludwigshafen (DE)**

(72) Erfinder : **Lauterbach, Gerald, Dr.**
**Arminstrasse 38a**
**W-6140 Bensheim (DE)**
Erfinder : **Pape, Frank-Friedrich, Dr.**
**Berner Weg 34**
**W-6700 Ludwigshafen (DE)**
Erfinder : **Gramlich, Walter, Dr.**
**Dammweg 4**
**W-6900 Heidelberg (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Substituierte Zimtaldehyde sind interessante Zwischenprodukte sowohl für die Synthese von Wirkstoffen als auch für die Darstellung von Riechstoffen. Von besonderem Interesse für die Riechstoffindustrie sind dabei die para-alkylsubstituierten Zimtaldehyde, da sie ideale Vorstufen für die als Riechstoffe begehrten entsprechenden Dihydrozimtaldehyde darstellen (vgl. GB 1086447). Die größte Bedeutung innerhalb dieser Substanzklasse besitzt der p-tert.-Butyl-dihydrozimtaldehyd, der als einer der wichtigsten Maiglöckchenriechstoffe in großen Mengen in der Parfümerie eingesetzt wird.

Nach dem bisherigen Stand der Technik werden alkylsubstituierte Zimtaldehyde - und hier insbesondere der p-tert.-Butyl-zimtaldehyd - durch Aldolreaktion aus den alkylsubstituierten Benzaldehyden und Acetaldehyd hergestellt (vgl. US 2, 976, 321). Um die Eigenkondensation des Acetaldehyds dabei möglichst gering zu halten und außerdem weitere Kondensationsreaktionen des entstehenden Zimtaldehyds mit Acetaldehyd zu minimieren, ist man gezwungen, die Reaktion mit erheblichem überschuß an dem kostspieligeren Reaktionspartner, dem substituierten Benzaldehyd, und in großer Verdünnung durchzuführen (vgl. US 2,976,321).

Ein weiteres Verfahren zur Herstellung von Zimtaldehyden wird in Zh. Org. Khim 1983, 19(4), Seiten 808-15 beschrieben. Bei diesem Verfahren werden die aus den entsprechenden Benzaldehyden gut zugänglichen Acetylenalkohole durch Umlagerung mit Poly(organovanadium)siloxanen in die gewünschten Zimtaldehyde überführt. Die Nachteile des genannten Verfahrens liegen insbesondere in der Verwendung kostspieliger und schwer zugänglicher Organovanadium-Silicium-Verbindungen und der mäßigen Ausbeute von maximal 65 %.

Gute Ausbeuten an unsubstituiertem Zimtaldehyd wurden gemäß C.A. 1951; 8997 g aus Phenylethinylcarbinol beim langsamen Zutropfen desselben in ein Gemisch aus Wasser, Schwefelsäure und einem mit Wasser mischbaren, keine alkoholischen OH-Gruppen enthaltenden Lösungsmittel, wie Dioxan oder Essigsäure erhalten. Nachteilig an diesem Verfahren ist, daß die Aufarbeitung des Reaktionsgemisches relativ aufwendig ist.

Es bestand daher die Aufgabe ein Verfahren zu finden, das die beschriebenen Nachteile vermeidet und alkylsubstituierte Zimtaldehyde in guter Ausbeute zugänglich macht.

Gegenstand der Erfindung ist ein verfahren zur Herstellung von alkylsubstituierten Zimtaldehyden der allgemeinen Formel I

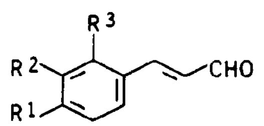

(I),

in der mindestens einer der Reste $R^1$, $R^2$ und $R^3$ für eine Alkylgruppe mit 1 bis 8 C-Atomen, vorzugsweise 1 bis 4 C-Atomen steht und die nicht für eine Alkylgruppe stehenden Reste $R^1$, $R^2$ oder $R^3$ Wasserstoff bedeuten, das dadurch gekennzeichnet ist, daß man Acetylenalkohole der allgemeinen Formel II

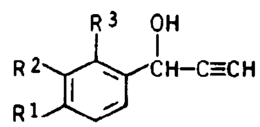

(II),

in der $R^1$, $R^2$ und $R^3$ die oben angegebene Bedeutung haben, in Gegenwart von 55 bis 98 %iger, vorzugsweise 70 bis 80 %iger wäßriger Ameisensäure erhitzt.

Aus Annales de Chimie 1967, Seiten 243-249, war zwar bereits die Umsetzung von Acetylenalkoholen der Formel

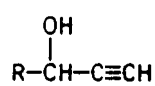

in Gegenwart von Ameisensäure oder dem stark sauren Ionenaustauscher Dowex 50 bekannt, jedoch wurden nur mit 2 von 12 der untersuchten Acetylenalkohole $\alpha,\beta$-ungesättigte Aldehyde erhalten - und dies auch nur in unbefriedigenden Ausbeuten. Bei der Umsetzung von Acetylenalkoholen, in denen R für einen aliphatischen oder cycloaliphatischen Rest stand, wurden lediglich Formiate oder Acetate gebildet. Bei der Umsetzung von

Acetylenalkoholen, in denen R für den o-Hydroxy-phenyl-, p-Methoxy-phenyl- oder den Furylrest standen, konnte nur Harzbildung beobachtet werden. Lediglich beim Einsatz von Acetylenalkoholen, in denen R für den Phenylrest oder den o-Chlor-phenylrest standen, wurden die entsprechenden Zimtaldehyde in Ausbeuten von 53 bis 60 % erhalten.

Dementsprechend war zu erwarten, daß auch bei der Umsetzung der Acetylenalkohole der allgemeinen Formel II mit sauren Katalysatoren wie Ameisensäure, Zimtaldehyde, wenn überhaupt, dann nur in schlechten Ausbeuten erhalten würden.

Ein weiteres Vorurteil gegen das vorteilhaft erfindungsgemäße Verfahren ergibt sich aus J. Amer. Chem. Soc. 70 (1948), Seite 3953. Hier heißt es, daß die von Rupe beschriebene Umlagerung von tertiären Acetylenalkoholen durch Kochen mit 85 %iger wäßriger Ameisensäure in die entsprechenden $\alpha,\beta$-ungesättigten Aldehyde bei Einsatz von aliphatischen oder cycloaliphatischen Acetylen-Alkoholen nicht bestätigt werden konnten.

Weiterhin heißt es, daß bei Einsatz von Phenyl-ethinylcarbinol zwar der Geruch von Zimtaldehyd auftrat, jedoch im wesentlichen Polymerisation zu teerigen Produkten eintrat.

In dem übersichtswerk Houben-Weyl, Methoden der Organischen Chemie, Georg-Thieme-Verlag, Stuttgart, New York, Band 6/1b, Seite 959 heißt es, daß der Verlauf der sogenannten Meyer-Schuster-Umlagerung

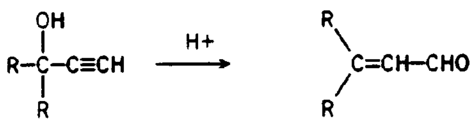


von Ethinylcarbinolen im einzelnen stark von den Substituenten sowie von dem angewandten sauren Reagenz - meist Ameisensäure oder Schwefelsäure - abhängt und daß Veresterungen, Dehydratisierungen und Spaltungsreaktionen häufig auftretende Konkurrenzreaktionen seien.

In Houben-Weyl, Band 7/1, Seite 110 heißt es, daß man lediglich bei Verwendung von Arylethinylcarbinolen durch Erhitzen mit verdünnten Säuren Zimtaldehyde erhält und zwar in Ausbeuten von ca. 30 %.

Dementsprechend war es sehr überraschend, daß man die alkylsubstituierten Zimtaldehyde der allgemeinen Formel I durch Erhitzen mit sauren Katalysatoren, insbesondere durch Erhitzen mit 55 bis 98 %iger, vorzugsweise 70 bis 80 %iger Ameisensäure in sehr guten Ausbeuten erhalten kann. Besonders überraschend war die Tatsache, daß man auch Acetylenalkohole mit den im allgemeinen als sehr säureempfindlich bekannten tertiären-Alkylgruppen im Benzolring, wie das p-tertiär-Butyl-phenyl-ethinyl-carbinol unter diesen drastischen Reaktionsbedingungen in 80 %iger Ausbeute in den gewünschten p-tert.-Butyl-zimtaldehyd überführen kann. Überraschenderweise wiesen die erhaltenen Zimtaldehyde der Formel I eine so hohe Reinheit auf, daß daraus beispielsweise eine problemlose Synthese der als Riechstoff begehrten empfindlichen alkylsubstituierten Dihydrozimtaldehyde möglich ist.

Als Acetylenalkohole der allgemeinen Formel II können beispielsweise eingesetzt werden:

p-tert.-Butyl-phenyl-ethinyl-carbinol, p-Isopropyl-phenyl-ethinyl-carbinol, p-Methyl-phenyl-ethinyl-carbinol.

Von besonderer Bedeutung ist die Umsetzung von p-tert.-Butylphenyl-ethinyl-carbinol und p-Isopropylphenyl-ethinyl-carbinol.

Das erfindungsgemäße Verfahren wird mit wäßriger Ameisensäure als saurem Katalysator durchgeführt. Die Konzentration der Ameisensäure kann in weiten Grenzen variiert werden. Bei Ameisensäurekonzentrationen unterhalb von etwa 55 bis 60 % beginnt der Umsatz zu sinken. Im allgemeinen kann die Ameisensäure in Konzentrationen von etwa 55 bis 98 % angewendet werden. Besonders vorteilhaft haben sich Konzentrationen von 70 bis 80 %, insbesondere von etwa 75 % erwiesen. Man verwendet sie im allgemeinen in Mengen von 200 bis 800, vorzugsweise etwa 400 bis 550 Gew.%, bezogen auf das Ethinylcarbinol der Formel II.

Das erfindungsgemäße Verfahren erlaubt eine kostengünstige und technisch einfach durchführbare Synthese der wichtigen alkylsubstituierten Zimtaldehyde der Formel I in hoher Reinheit. Die zur Umlagerung verwendete Ameisensäure kann problemlos recyclisiert werden, indem man das Reaktionsgemisch mit einem Lösungsmittel, wie Toluol, extrahiert und die nach der Phasentrennung zurückgewonnene Säure erneut zur Umlagerung einsetzt. Hierdurch ist gewährleistet, daß bei diesem Verfahren ein Minimum an Abfallprodukten anfällt. Durch einfache Destillation der organischen Phase werden die alkylsubstituierten Zimtaldehyde in großer Reinheit gewonnen.

Beispiel 1

Ein Gemisch aus 250 g 75 %iger wäßriger Ameisensäure, 50 g p-tert.-Butyl-phenyl-ethinylcarbinol und 0,5 g Hydrochinon (als Antioxidans) wird eine Stunde unter Rückfluß zum Sieden erhitzt. Nach dem Abkühlen des Reaktionsgemisches extrahierte man zweimal mit je 250 ml Toluol. Die wäßrige Phase wurde abgetrennt und bei dem nächsten Ansatz wieder eingesetzt. Die organische Phase wurde nach dem Waschen mit $NaHCO_3$-Lösung eingeengt und anschließend destilliert. Man erhielt 40,5g p-tert.-Butyl-zimtaldehyd mit einem Siedepunkt von 95 bis 102°C/002 mbar (entsprechend einer Ausbeute von 80 % d.Th.).

Beispiel 2

Durch 1-stündiges Erhitzen eines Gemisches aus 250 g einer 75 %igen wäßrigen Ameisensäure, 50 g p-Isopropyl-phenyl-ethinylcarbinol und 0,5 g Hydrochinon unter Rückfluß und anschließende Aufarbeitung des Reaktionsgemisches analog Beispiel 1 erhielt man 38 g (entsprechend 76 % der Theorie) p-Isopropyl-zimtaldehyd mit einem Siedepunkt von 90 bis 95°C/0,06 mbar.

Beispiel 3

Durch 1-stündiges Erhitzen eines Gemisches aus 250 g einer 75 %igen wäßrigen Ameisensäure, 50 g p-Methyl-phenyl-ethinylcarbinol und 0,5g Hydrochinon unter Rückfluß und anschließende Aufarbeitung analog Beispiel 1 erhielt man 40 g (entsprechend 80 % d.Theorie) an p-Methyl-Zimtaldehyd mit einem Siedepunkt von 90 bis 95°C/0,1 mbar.

## Patentansprüche

1. Verfahren zur Herstellung von alkylsubstituierten Zimtaldehyden der allgemeinen Formel I

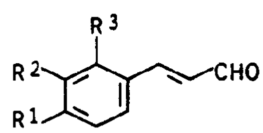

(I),

in der mindestens einer der Reste $R^1$, $R^2$ und $R^3$ für eine Alkylgruppe mit 1 bis 8 C-Atomen und die nicht für eine Alkylgruppe stehenden Reste $R^1$, $R^2$ oder $R^3$ Wasserstoff bedeuten, das dadurch gekennzeichnet ist, daß man Acetylenalkohole der allgemeinen Formel II

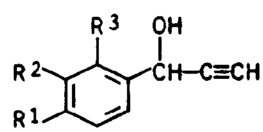

(II),

in der $R^1$, $R^2$ und $R^3$ die oben angegebene Bedeutung haben, in Gegenwart von 55 bis 98 %iger wäßriger Ameisensäure erhitzt.

2. Verfahren gemäß Anspruch 1, dadurch gekennzeichnet, daß man in Gegenwart von 70 bis 80 %iger wäßriger Ameisensäure erhitzt.

## Claims

1. A process for preparing an alkyl-substituted cinnamaldehyde of the general formula I

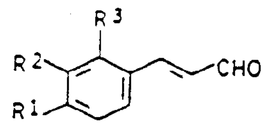

(I)

where one or more of $R^1$, $R^2$ and $R^3$ is alkyl of from 1 to 8 carbon atoms and non-alkyl $R^1$, $R^2$ and $R^3$ are each hydrogen, which comprises heating an acetylene alcohol of the general formula II

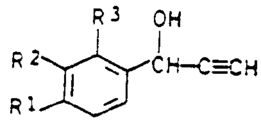

(II)

where $R^1$, $R^2$ and $R^3$ are each as defined above, in the presence of from 55 to 98 % strength aqueous formic acid.

**2.** A process as claimed in claim 1, wherein the heating is carried out in the presence of from 70 to 80 % strength aqueous formic acid.

## Revendications

1. - Procédé de préparation d'aldéhydes cinnamiques alkylsubstitués de formule générale I

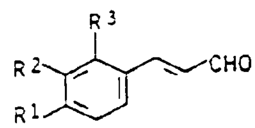

(I),

dans laquelle l'un au moins des restes $R^1$, $R^2$ et $R^3$ est mis pour un groupement alkyle à 1-8 atomes de carbone et les restes $R^1$, $R^2$ ou $R^3$ qui ne sont pas mis pour un groupement alkyle représentent des atomes d'hydrogène, caractérisé en ce qu'on chauffe des alcools acétyléniques de formule générale II

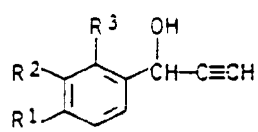

(II),

dans laquelle $R^1$, $R^2$ et $R^3$ ont les significations données ci-dessus, en présence d'acide formique aqueux à 55-98%.

2. - Procédé selon la revendication 1, caractérisé en ce qu'on chauffe en présence d'acide formique aqueux à 70-80%.